(19) Europäisches Patentamt
European Patent Office
Office européen des brevets

(11) EP 3 064 237 A1

(12) EUROPEAN PATENT APPLICATION
published in accordance with Art. 153(4) EPC

(43) Date of publication:
**07.09.2016 Bulletin 2016/36**

(21) Application number: **14883669.5**

(22) Date of filing: **28.02.2014**

(51) Int Cl.:
***A61M 5/24*** *(2006.01)* ***A61M 5/20*** *(2006.01)*

(86) International application number:
**PCT/KR2014/001657**

(87) International publication number:
**WO 2015/129940 (03.09.2015 Gazette 2015/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Choi, Kyu Dong**
**Seoul 153-797 (KR)**

(72) Inventor: **Choi, Kyu Dong**
**Seoul 153-797 (KR)**

(74) Representative: **chapman + co**
**Senghennydd Road**
**Cardiff, South Wales CF24 4AY (GB)**

# (54) DEVICE AND METHOD FOR INJECTING DRUG

(57) Disclosed herein is a method for injecting a medication, including injecting the medication into a subject by pushing the medication filled in a cartridge with a pusher, and moving the pusher backwards to a predetermined distance in a direction opposite to a direction of pushing the pusher when a prescribed dose of medication has been injected.

Fig. 1

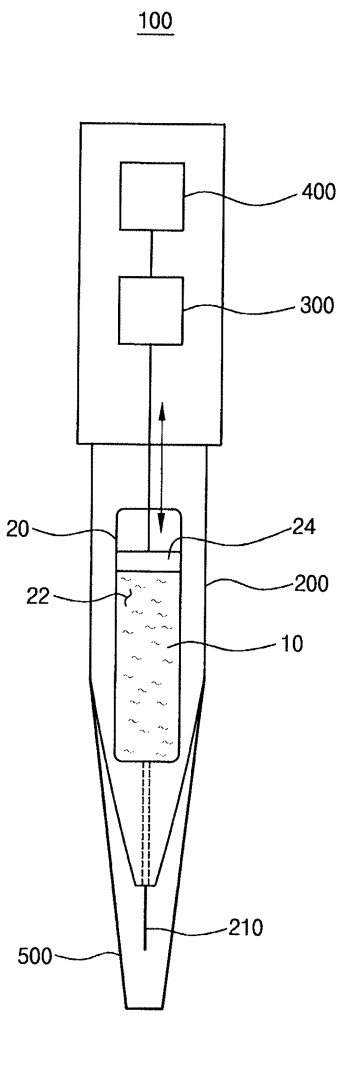


EP 3 064 237 A1

**Description**

BACKGROUND OF THE INVENTION

Exemplary embodiments of the present invention relate to an apparatus and method for injecting medication, and more particularly, to a medication injecting apparatus capable of injecting medication while carrying the medication, such as insulin, which is required to precisely adjust an injection dose thereof, and to a medication injecting method using the medication injecting apparatus.

Generally, the type of insulin used to treat diabetic metabolic disease may be classified into mealtime insulin and basal insulin. The meal insulin is usually injected into a body three times a day when a diabetic is in a fasting state before meals to prevent the blood sugar of a diabetic from sharply rising after meal. The basal insulin is insulin that continuously and steadily acts on the body for 24 hours.

Diabetes is a medical condition in which the body does not produce enough insulin or does not use insulin properly, so that the blood sugar, namely glucose, is not converted into energy. Diabetes is divided into type 1 diabetes and type 2 diabetes.

Type 1 diabetes develops when the immune system mistakes beta cells producing insulin in the pancreas for invaders and attacks the beta cells, and usually its symptoms appear suddenly. This is mostly diagnosed in children and is referred to as juvenile type diabetes. However, type 1 diabetes may occur even in adults.

Type 2 diabetes occurs when a body loses the function of using insulin properly even if the beta cells produce enough insulin. This shows symptoms slowly, is mostly diagnosed in the middle-aged and thus is called adult type diabetes. However, overweight children may have type 2 diabetes.

Diabetics use a portable injecting apparatus to inject themselves with insulin that is medication at any time in daily life. For this reason, a portable injecting apparatus includes a cartridge installing unit. A cartridge is installed in the cartridge installing unit, and has a receiving space filled with insulin and a pusher installed to push the insulin while sealing the insulin in the receiving space. The cartridge installing unit includes a discharge part through which the insulin is substantially discharged.

Since the pusher is made of an elastic material, such as rubber, to seal the insulin, an additional dose of insulin may be discharged due to the deformation of the pusher after a prescribed dose of insulin is injected into a patient by pushing the insulin via the pusher. This may cause the unnecessary waste of insulin and create wet environment in a space around the discharge part, so that germs may propagate more easily. The propagation of the germs may have a lethal effect on diabetics who are susceptible to complications.

SUMMARY OF THE INVENTION

An object of the present invention is to provide a medication injecting method, which is capable of preventing medication from being further discharged due to the deformation of a pusher after a prescribed dose of medication, such as insulin, is injected into a patient who is a subject.

Another object of the present invention is to provide a medication injecting apparatus to which the above-described method is applied.

In order to accomplish an object of the present invention, in accordance with an aspect of the present invention, provided is a method for injecting a medication, including injecting the medication into a subject by pushing the medication filled in a cartridge with a pusher and moving the pusher backwards to a predetermined distance in a direction opposite to a direction of pushing the pusher when a prescribed dose of medication has been injected.

According to an exemplary embodiment, the predetermined distance may be defined by following Equation 1:

Equation 1

$$predetermined\ distance \leq \frac{\text{torque } of\ pushing\ driving\ unit \times moving\ distance\ of\ pusher}{\text{internal sectional area of cartridge} \times \text{elastic modulus of pusher}}$$

In order to accomplish another object of the present invention, in accordance with another aspect of the present invention, an apparatus for injecting a medication may include a cartridge installing unit, a driving unit, and a drive control unit. A cartridge may be installed in the cartridge installing unit and may have a receiving space in which the medication may be filled, and a pusher which may be mounted to push the medication while sealing the medication in the receiving space, and the cartridge installing unit may have on one end thereof a discharge part that may discharge the medication from the cartridge to inject the medication into the subject. The driving unit may be connected with the pusher, and may provide a driving force to the pusher to allow the pusher to push the medication. The drive control unit may be connected with the driving unit, and may control the driving unit to move the pusher backwards to a predetermined distance in a direction opposite to a direction of pushing the pusher when a prescribed dose of medication has been injected.

According to an exemplary embodiment, the predetermined distance may be defined by following Equation 2:

Equation 2

$$\mathit{predetermined\ distance} \leq \frac{\text{torque of } \mathit{driving\ unit} \times \mathit{moving\ distance\ of\ pusher}}{\text{internal sectional area of cartridge} \times \text{elastic modulus of pusher}}$$

According to an exemplary embodiment, the driving unit may include a motor generating a rotating force, and a power transmission part connected between the motor and the pusher and precisely transmitting a driving force to the pusher while being rotated in a screw form by the rotating force of the motor.

According to an exemplary embodiment, the medication injecting apparatus may further include a cover coupled to the cartridge installing unit while covering the discharge part.

The medication injecting apparatus and method are advantageous in that the medication filled in the cartridge is pushed by the pusher configured to seal the medication to be injected into a subject who is a patient, and then the pusher is moved backwards to a predetermined distance in a direction opposite to a direction of pushing the pusher when a prescribed dose of medication has been injected, so that, even if the pusher made of the elastic material to seal the medication is deformed, the deformation may be compensated for by moving the pusher backward to the predetermined distance. Therefore, the invention is capable of preventing an excessive dose of medication due to the deformation of the pusher from being discharged, thus preventing the waste of the medication.

Further, the invention prevents the surroundings of the discharge part through which the medication is substantially discharged from creating wet environment, due to a surplus dose of medication, thus preventing germs from easily propagating. Therefore, this can safely protect a patient from lethal disease caused by the germs.

## BRIEF DESCRIPTION OF THE DRAWINGS

FIG. 1 is a view schematically illustrating the configuration of a medication injecting apparatus according to an embodiment of the present invention;

FIG. 2 is a view illustrating an interior of a cartridge of the medication injecting apparatus illustrated in FIG. 1;

FIG. 3 is a view schematically illustrating a state in which a driving force is exerted on a pusher in the cartridge illustrated in FIG. 2; and

FIG. 4 is a flowchart illustrating a method for injecting medication through the pusher in the cartridge illustrated in FIG. 2.

## DETAILED DESCRIPTION OF PREFERRED EMBODIMENTS

A method for injecting a medication according to the present invention includes injecting the medication into a subject by pushing the medication filled in a cartridge with a pusher, and then moving the pusher backwards to a predetermined distance in a direction opposite to a direction of pushing the pusher when a prescribed dose of medication has been injected.

According to an exemplary embodiment, the predetermined distance may be defined by following Equation 1:

Equation 1

$$\mathit{predetermined\ distance} \leq \frac{\text{torque } \mathit{of\ pushing\ driving\ unit} \times \mathit{moving\ distance\ of\ pusher}}{\text{internal sectional area of cartridge} \times \text{elastic modulus of pusher}}$$

An apparatus for injecting a medication according to the present invention includes a cartridge installing unit in which a cartridge is installed, the cartridge having a receiving space in which the medication is filled, and a pusher mounted to push the medication while sealing the medication in the receiving space, the cartridge installing unit having on one end thereof a discharge part that discharges the medication from the cartridge to inject the medication into the subject; a driving unit connected with the pusher and providing a driving force to the pusher to allow the pusher to push the medication; and a drive control unit connected with the driving unit and controlling the driving unit to move the pusher backwards to a predetermined distance in a direction opposite to a direction of pushing the pusher when a prescribed dose of medication has been injected.

According to an exemplary embodiment, the predetermined distance of the backward movement may be defined by following Equation 2:

Equation 2

$$predetermined\ distance \leq \frac{\text{torque of } driving\ unit \times moving\ distance\ of\ pusher}{\text{internal sectional area of cartridge} \times \text{elastic modulus of pusher}}$$

Hereinafter, an apparatus and method for injecting medication according to an exemplary embodiment of the present invention will be described in detail with reference to the accompanying drawings. The invention may have various modifications and alternative forms, and particular embodiments are illustrated by way of examples in the drawings and will be described herein in detail. It should be understood, however, that there is no intention to limit exemplary embodiments of the present invention. On the contrary, the exemplary embodiments are to cover all modifications, equivalents and substitutes. Like reference numerals are used to identify like elements throughout the drawings. For the clarity of description, the dimensions of the elements are exaggerated in the drawings.

Although the terms "first", "second", etc. are used herein to describe various elements, these elements should not be limited by these terms. These terms are only used to distinguish one element from another element. For example, the first element may be designated as the second element, and the second element may be likewise designated as the first element without departing from the scope of the invention.

It is to be understood that terms employed herein are for the purpose of description of particular embodiments and not of limitation. The singular forms "a" and "an" include plural referents unless otherwise stated. Further, it should be understood that terms "include" or " have" are inclusive of characteristics, numerals, steps, operations, elements, parts or combination thereof, which are described herein, bur are not exclusive of one or more different characteristics, numerals, steps, operations, elements, parts or combination thereof.

Unless otherwise defined, all terms including technical and scientific terms used herein have the same meaning as commonly understood by one of ordinary skill in the art to which the present invention belongs. It will be further understood that terms defined in commonly used dictionaries should be interpreted as having a meaning that is consistent with their meaning in the context of the relevant art and will not be interpreted in an idealized or overly formal sense unless expressly so defined herein.

FIG. 1 is a view schematically illustrating the configuration of a medication injecting apparatus according to an embodiment of the present invention, FIG. 2 is a view illustrating an interior of a cartridge of the medication injecting apparatus illustrated in FIG. 1, and FIG. 3 is a view schematically illustrating a state in which a driving force is exerted on a pusher in the cartridge illustrated in FIG. 2.

Referring to FIGS. 1 to 3, a medication injecting apparatus 100 according to an exemplary embodiment of the present invention includes a cartridge installing unit 200, a driving unit 300, and a drive control unit 400.

A cartridge 20 is installed in the cartridge installing unit 200. The cartridge 20 includes a receiving space 22 in which a medication 10 is filled, and a pusher 24 that pushes the medication 10 while sealing the medication 10 in the receiving space 22. Such a cartridge 20 may have a structure similar to that of a syringe in which a piston is installed.

To be more specific, the pusher 24 configured to push the medication 10 in the receiving space 22 defined in the cartridge 20 may be made of an elastic material, such as rubber or silicon, so as to perfectly seal the medication 10. Meanwhile, examples of the medication 10 may include insulin that is absolutely required for diabetics to preserve their lives. In addition, examples of the medication 10 may include other medication, such as a Botox injection, which should be precisely controlled in a beauty care or a cosmetic surgery.

Further, the cartridge installing unit 200 has on one end thereof a discharge part 210 that is connected to the cartridge 20 to discharge the medication 10. Thus, the discharge part 210 is a part that substantially discharges the medication 10 filled in the cartridge 20, and may have the shape of a needle to allow the medication 10 to be injected into a subject. In this regard, it may be understood that, if the medication 10 includes insulin or a Botox injection, the subject is limited to a patient to who requires the injection of the medication 10.

The driving unit 300 is connected with the pusher 24. The driving unit 300 provides a driving force to the pusher 24 to allow it to push the medication 10, thus discharging the medication 10 through the discharge part 210. If the medication 10 includes insulin or a Botox injection, particularly insulin that causes shock due to low blood sugar when an excessive dose of insulin is injected, in order to precisely control a discharge amount thereof, the driving unit 300 may include a motor 310 that generates a rotating force, and a power transmission part 320 that precisely converts the rotating force into a linear driving force to thereby transmit the linear driving force to the pusher 24, instead of using a cylinder that directly generates a linear driving force.

To be more specific, the power transmission part 320 is connected between the motor 310 and the pusher 24 to transmit the linear driving force to the pusher 24 while being rotated in a screw form via the rotating force of the motor 310. At this time, the driving unit 300 may more precisely control a moving distance of the pusher 24 by adjusting an angle of the power transmission part 320 in the screw form.

The drive control unit 400 is connected with the driving unit 300. To be more specific, the drive control unit 400 may control the discharge of the medication 10 from the discharge part 210, by supplying or interrupting driving power to the motor 310 of the driving unit 300. Further, the drive control unit 400 may control the discharge amount of the medication 10, by controlling voltage of the driving power and hence the rotating force of the motor 310.

The medication injecting apparatus 100 configured as such may have the shape of a pen that is convenient to carry, as illustrated in FIG. 1, so as to allow the medication 10 to be easily injected anywhere and at any time. In this case, a battery (not shown) may be installed in the drive control unit 400 to provide the driving power to the motor 310. Preferably, the battery comprises a secondary battery that is rechargeable to ensure the easy use of the medication injecting apparatus 100. However, in the case of an emergency situation, for example, in the case where it is required to inject insulin to a diabetic, the medication injecting apparatus may use electricity of a battery of a mobile phone that is generally carried by people. To this end, the drive control unit 400 may be provided with a connecting jack (not shown) that is electrically connectable to the battery of the mobile phone.

According to an exemplary embodiment of the present invention, the medication injecting apparatus 100 may further include a medication reservoir (not shown), a communication line, and a valve (not shown). The medication reservoir communicates with the cartridge 20 to feed the medication to the receiving space 22 of the cartridge 20. The communication line makes the cartridge 20 and the medication reservoir communicate with each other. The valve is provided on the communication line to open or close the communication line.

Hereinafter, a method for substantially injecting the medication 10 using the medication injecting apparatus 100 will be described in more detail by further referring to FIG. 4.

FIG. 4 is a flowchart illustrating the method for injecting the medication through the pusher in the cartridge illustrated in FIG. 2.

Referring to FIG. 4, first, the discharge part 210 of the cartridge installing unit 200 is inserted into a patient who is a subject, at step S100.

Subsequently, the medication 10 filled in the cartridge 20 is pushed by the pusher 24 through the linear driving force transmitted from the power transmission part 320 of the driving unit 300 to be injected into the patient, at step S200. In this case, a static frictional force having a first magnitude is first exerted between the pusher 24 and an inner wall of the cartridge 20, and then a dynamic frictional force having a second magnitude that is smaller than the first magnitude is exerted while the pusher 24 pushing the medication 10. Thereafter, while the pusher 24 pushes the medication 10, a lubrication frictional force having a third magnitude that is smaller than the second magnitude is exerted by lubrication action of the medication 10. At this time, if the lubrication frictional force of the smallest third magnitude is exerted and then the pusher 24 is stopped, the pusher 24 may be deformed in the direction of the medication 10 by the action of the static frictional force having the first magnitude. This means that the medication 10 may be further pushed due to the deformation of the pusher even if the pusher 24 is stopped.

Subsequently, at a time when a prescribed dose of medication 10 is injected, specifically at a time when a required dose is injected into a patient, the motor 310 of the driving unit 300 is rotated reverse by the drive control unit 400 to move the pusher 24 backwards to a predetermined distance PB in a direction opposite to the pushing direction, at step S300.

This prevents the medication 10 from being further pushed due to the above-mentioned deformation of the pusher 24, thus preventing an excessive dose of medication 10 from being discharged from the discharge part 210. In this way, it is possible to prevent the medication 10 from being wasted, so that the service life of the cartridge 20 may be maximized.

In this case, the predetermined distance PB to which the pusher 24 is moved backwards may be defined by the following Equation.

[Equation]

$$predetermined\ distance\ (PB) \leq \frac{\text{torque } (\tau)\ of\ driving\ unit \times moving\ distance\ (PF)}{\text{internal sectional area (A) of cartridge} \times \text{elastic modulus of pusher}}$$

According to the above Equation, the predetermined distance PB may be equal to or less than a value that is in inverse proportion to an internal sectional area A of the cartridge 20 and an elastic modulus of the pusher 24 and is in proportion to torque $\tau$ of the driving unit 300 and a moving distance PF of the pusher 24. Here, the torque $\tau$ of the driving unit 300 corresponds to the torque of the motor 310 included in the driving unit 300.

The elastic modulus of the pusher 24 is a value that is generally fixed according to the material, and may have the value of about 2 MPa in the case of rubber. Thus, for example, if the pusher 24 has the elastic modulus that is smaller than that of the rubber, namely, if the pusher 24 is more easily deformed, the predetermined distance PB may be increased in inverse proportion.

Thereafter, in the state where the pusher 24 is moved backwards to the predetermined distance PB, the discharge part 210 is separated from the patient. Such a configuration may prevent the discharge part 210 from being separated from the patient when a prescribed dose of medication 10 is injected by the pusher 24, that is, before the pusher 24 is moved backwards, thus perfectly preventing an excessive dose of medication 10 from being discharged and injected into the patient.

In order to protect the needle-shaped discharge part 210 from an outside, the medication injecting apparatus 100 may further include a cover 500 that is coupled to the cartridge installing unit 200 while covering the discharge part 210. In this case, the backward movement of the pusher 24 prevents an excessive dose of medication 10 from being discharged from the discharge part 210, so that it is possible to prevent a wet environment from being created in the surroundings of the discharge part 210 that are an internal space of the cover 500. Therefore, it is possible to prevent germs from easily propagating in the internal space of the cover 500, thus safely protecting a patient from lethal diseases caused by the germs. Particularly, in the case where the medication 10 is insulin that is injected into a diabetic, the suppression of the germ propagation may be more effective for the diabetic who may be died of complications.

While the present invention has been described with respect to the specific embodiments, it will be apparent to those skilled in the art that various changes and modifications may be made without departing from the spirit and scope of the invention as defined in the following claims.

As described above, an exemplary embodiment of the present invention provides an apparatus and method for injecting medication, in which a medication filled in a cartridge is pushed by a pusher configured to seal the medication to be injected into a subject who is a patient, and then the pusher is moved backwards to a predetermined distance in a direction opposite to a direction of pushing the pusher when a prescribed dose of medication has been injected, so that, even if the pusher made of an elastic material to seal the medication is deformed, the deformation may be compensated for by moving the pusher backward to the predetermined distance. Consequently, the invention is capable of preventing the waste of the medication.

## Claims

1. A method for injecting a medication, comprising:

   injecting the medication into a subject by pushing the medication filled in a cartridge with a pusher; and moving the pusher backwards to a predetermined distance in a direction opposite to a direction of pushing the pusher when a prescribed dose of medication has been injected.

2. The method according to claim 1, wherein the predetermined distance is defined by following Equation 1:

   Equation 1

$$predetermined\ distance \leq \frac{\text{torque } of\ pus\ \ ing\ driving\ unit \times moving\ distance\ of\ pus\ \ er}{\text{internal sectional area of cartridge} \times \text{elastic modulus of pusher}}$$

3. An apparatus for injecting a medication, comprising:

a cartridge installing unit in which a cartridge is installed, the cartridge having a receiving space in which the medication is filled, and a pusher mounted to push the medication while sealing the medication in the receiving space, the cartridge installing unit having on one end thereof a discharge part that discharges the medication from the cartridge to inject the medication into the subject;
a driving unit connected with the pusher, and providing a driving force to the pusher to allow the pusher to push the medication; and
a drive control unit connected with the driving unit, and controlling the driving unit to move the pusher backwards to a predetermined distance in a direction opposite to a direction of pushing the pusher when a prescribed dose of medication has been injected.

**4.** The apparatus according to claim 3, wherein the predetermined distance is defined by following Equation 2:

Equation 2

$$predetermined\ distance \le \frac{\text{torque o}f\ driving\ unit\ \times moving\ distance\ of\ pus\ \ er}{\text{internal sectional area of cartridge } \times \text{ elastic modulus of pusher}}$$

**5.** The apparatus according to claim 3, wherein the driving unit comprises:

a motor generating a rotating force; and
a power transmission part connected between the motor and the pusher, and transmitting a driving force to the pusher while being rotated in a screw form by the rotating force of the motor.

**6.** The apparatus according to claim 3, further comprising:

a cover coupled to the cartridge installing unit while covering the discharge part.

Fig. 1

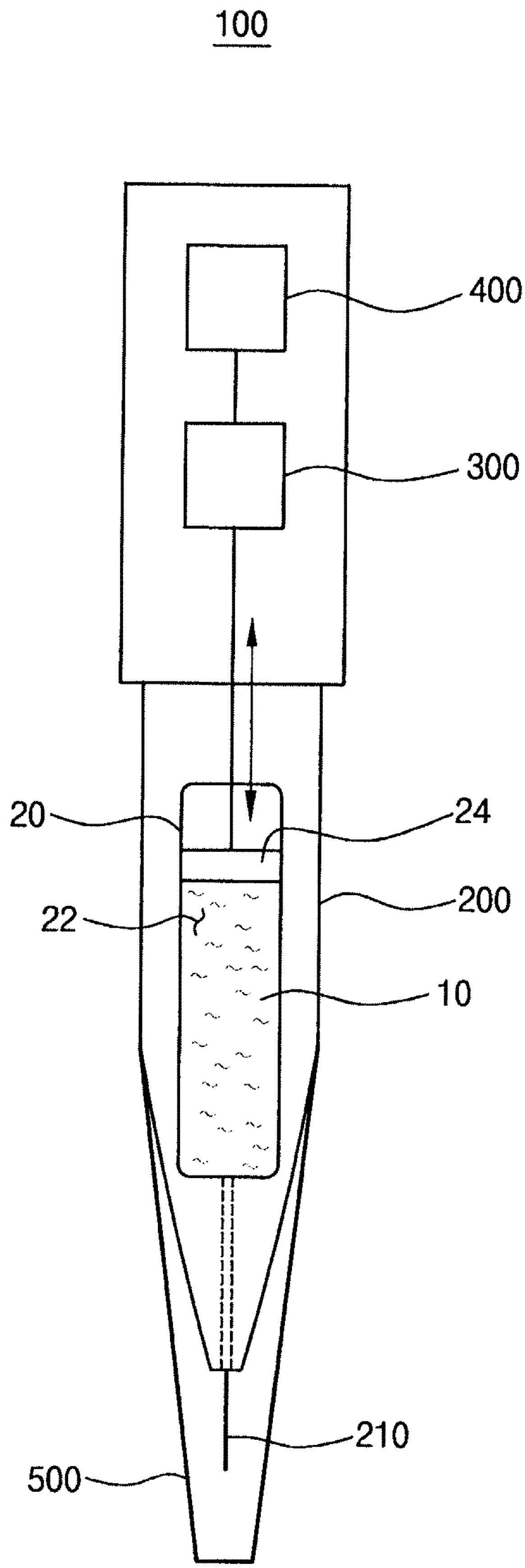

Fig. 2

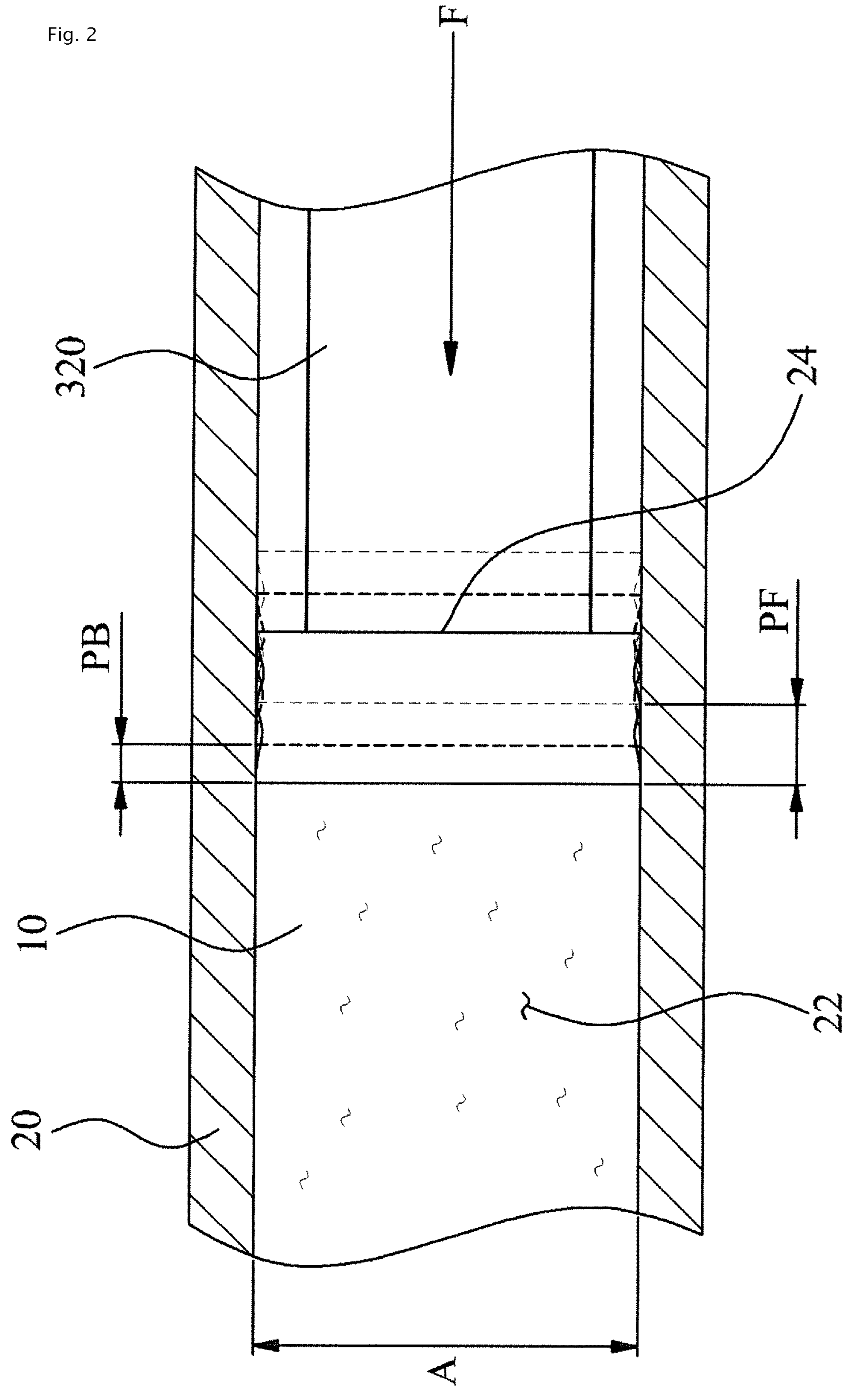

Fig. 3

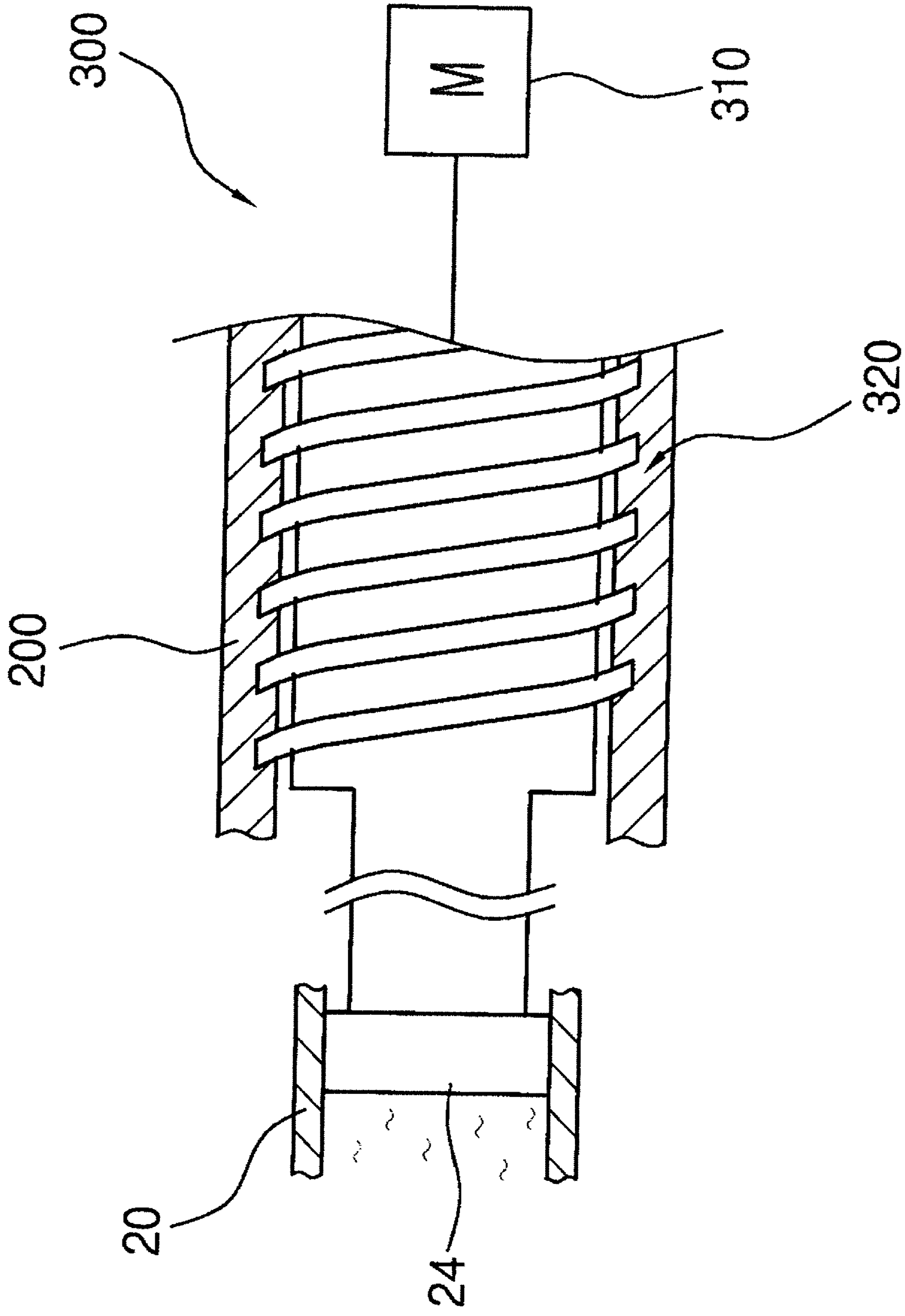

Fig. 4

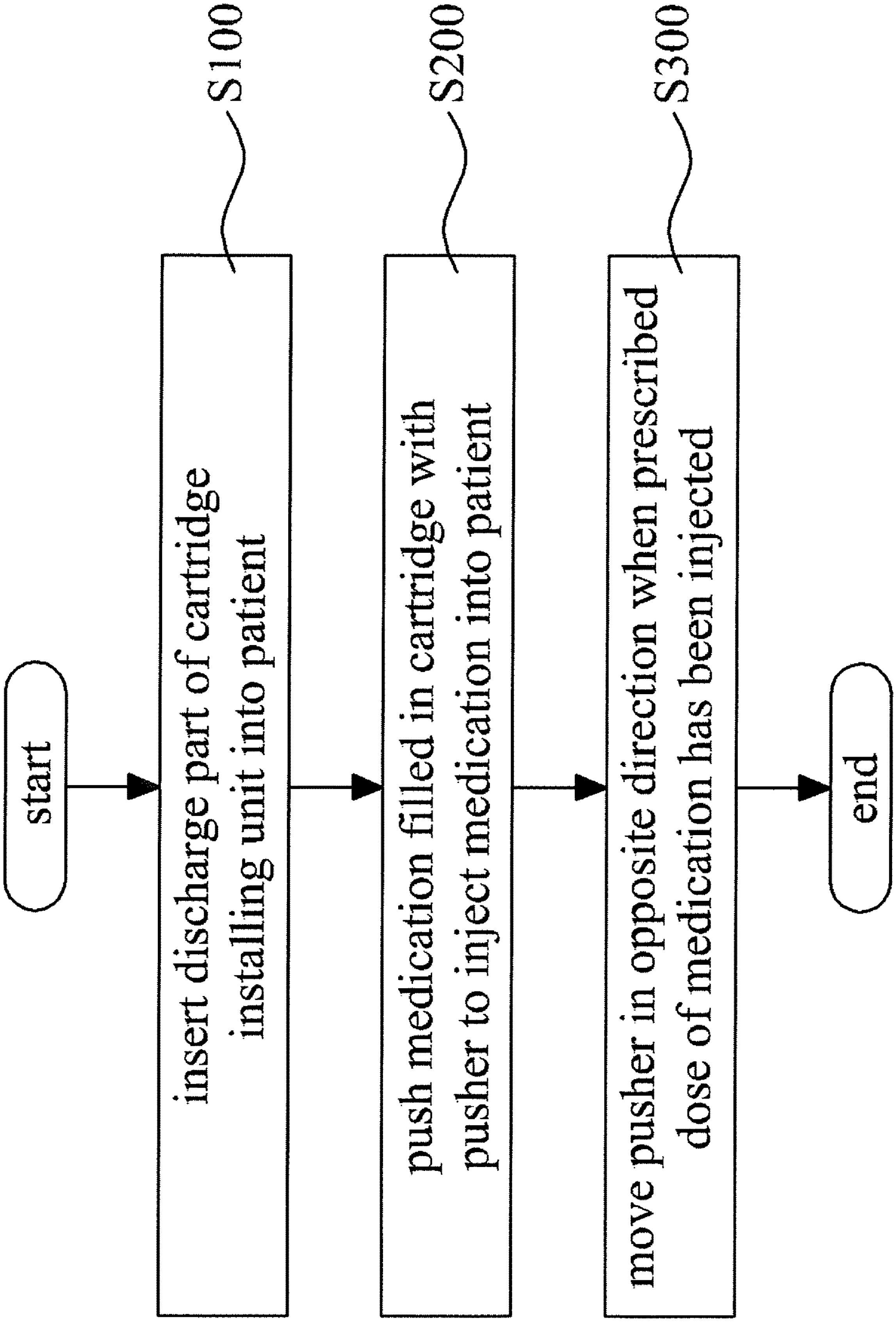
start
insert discharge part of cartridge installing unit into patient
S100
push medication filled in cartridge with pusher to inject medication into patient
S200
move pusher in opposite direction when prescribed dose of medication has been injected
S300
end

# INTERNATIONAL SEARCH REPORT

International application No.
**PCT/KR2014/001657**

A. CLASSIFICATION OF SUBJECT MATTER

***A61M 5/24(2006.01)i, A61M 5/20(2006.01)i***

According to International Patent Classification (IPC) or to both national classification and IPC

B. FIELDS SEARCHED

Minimum documentation searched (classification system followed by classification symbols)

A61M 5/24; A61M 5/145; A61M 5/48; A61M 5/00; A61M 5/20; A61M 5/168

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: injection device, infusion pump, cartridge, plunger, needle, drug, motor, pusher, torque, elastic modulus, reverse rotation, retreat, constant volume.

C. DOCUMENTS CONSIDERED TO BE RELEVANT

| Category* | Citation of document, with indication, where appropriate, of the relevant passages | Relevant to claim No. |
|---|---|---|
| X<br>A | EP 1240913 A2 (NIPRO CORPORATION) 18 September 2002<br>See abstract, paragraphs [0014], [0015], [0026], figures 1, 3, 5 | 3,5,6<br>4 |
| A | WO 98-00188 A1 (PHARMACIA & UPJOHN AB) 08 January 1998<br>See the entire document | 3-6 |
| A | US 2005-0038389 A1 (Frank, M. Fago et al.) 17 February 2005<br>See the entire document | 3-6 |
| A | US 2011-0224644 A1 (HAUETER, Ulrich et al.) 15 September 2011<br>See the entire document | 3-6 |
| A | US 2009-0124974 A1 (CRANK, Justin M. et al.) 14 May 2009<br>See the entire document | 3-6 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 NOVEMBER 2014 (19.11.2014) | **19 NOVEMBER 2014 (19.11.2014)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

INTERNATIONAL SEARCH REPORT

International application No.
**PCT/KR2014/001657**

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **1, 2**
because they relate to subject matter not required to be searched by this Authority, namely:
Claims 1 and 2 pertain to a method for treatment of the human body by surgery or therapy, and thus pertain to subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**
Information on patent family members

International application No.
**PCT/KR2014/001657**

| Patent document cited in search report | Publication date | Patent family member | Publication date |
|---|---|---|---|
| EP 1240913 A2 | 18/09/2002 | DE 60202088 D1 | 05/01/2005 |
| | | DE 60202088 T2 | 01/12/2005 |
| | | DK 1240913 T3 | 04/04/2005 |
| | | EP 1240913 A3 | 11/12/2002 |
| | | EP 1240913 B1 | 01/12/2004 |
| | | JP 03845857 B2 | 15/11/2006 |
| | | JP 2002-272839 A | 24/09/2002 |
| | | US 2002-0133114 A1 | 19/09/2002 |
| | | US 6913591 B2 | 05/07/2005 |
| WO 98-00188 A1 | 08/01/1998 | AT 233111 T | 15/03/2003 |
| | | AU 1997-34689 B2 | 09/12/1999 |
| | | AU 3468997 A | 21/01/1998 |
| | | AU 713846 B2 | 09/12/1999 |
| | | BR 9710121 A | 10/08/1999 |
| | | CA 2259442 A1 | 08/01/1998 |
| | | CA 2259442 C | 06/06/2006 |
| | | CN 1176727 C0 | 24/11/2004 |
| | | CN 1224364 A0 | 28/07/1999 |
| | | CN 1589916 A | 09/03/2005 |
| | | CN 1589916 C0 | 09/03/2005 |
| | | CZ 294265 B6 | 10/11/2004 |
| | | CZ 9804380 A3 | 16/06/1999 |
| | | DE 69719335 D1 | 03/04/2003 |
| | | DE 69719335 T2 | 16/10/2003 |
| | | DK 0921828 T3 | 16/06/2003 |
| | | EP 0921828 B1 | 26/02/2003 |
| | | ES 2193383 T3 | 01/11/2003 |
| | | HU 0000104 A2 | 28/06/2000 |
| | | HU 0000104 A3 | 28/07/2000 |
| | | IL 127618 A | 05/09/2006 |
| | | IL 127618 D0 | 28/10/1999 |
| | | JP 2000-513261 A | 10/10/2000 |
| | | JP 2000-513261 T | 10/10/2000 |
| | | KR 10-0383477 B1 | 22/09/2003 |
| | | KR 10-2000-0022473 A | 25/04/2000 |
| | | NO 316312 B1 | 12/01/2004 |
| | | NO 986206 A | 11/02/1999 |
| | | NO 986206 D0 | 30/12/1998 |
| | | PL 185530 B1 | 30/05/2003 |
| | | PL 330845 A1 | 07/06/1999 |
| | | PT 921828 E | 31/07/2003 |
| | | PT 921828 T | 31/07/2003 |
| | | TW 486375 A | 11/05/2002 |
| | | TW 486375 B | 11/05/2002 |
| | | US 06042571 A | 28/03/2000 |
| | | WO 98-00188A1 | 08/01/1998 |
| US 2005-0038389 A1 | 17/02/2005 | AU 2003-261213 A1 | 23/02/2004 |

INTERNATIONAL SEARCH REPORT
Information on patent family members

International application No.
PCT/KR2014/001657

| Patent document cited in search report | Publication date | Patent family member | Publication date |
|---|---|---|---|
| | | AU 2003-261213 B2 | 24/07/2008 |
| | | AU 2008-229826 A1 | 30/10/2008 |
| | | AU 2008-229826 B2 | 12/05/2011 |
| | | BR 0313205 A | 28/06/2005 |
| | | BR 0313205 B1 | 05/02/2013 |
| | | CA 2494553 A1 | 12/02/2004 |
| | | CA 2494553 C | 19/04/2011 |
| | | CA 2730216 A1 | 12/02/2004 |
| | | CA 2730216 C | 21/01/2014 |
| | | CA 2730219 A1 | 12/02/2004 |
| | | CN 100411696 C0 | 20/08/2008 |
| | | CN 101352590 A | 28/01/2009 |
| | | CN 101352590 B | 14/09/2011 |
| | | CN 101352591 A | 28/01/2009 |
| | | CN 101352591 B | 18/07/2012 |
| | | CN 101352592 A | 28/01/2009 |
| | | CN 101352592 B | 27/07/2011 |
| | | CN 1684729 A | 19/10/2005 |
| | | CN 1684729 C0 | 20/08/2008 |
| | | EP 1526881 A2 | 04/05/2005 |
| | | EP 1526881 A4 | 28/11/2007 |
| | | EP 2359884 A2 | 24/08/2011 |
| | | EP 2359884 A3 | 28/12/2011 |
| | | EP 2609949 A1 | 03/07/2013 |
| | | JP 05085617 B2 | 28/11/2012 |
| | | JP 05564446 B2 | 30/07/2014 |
| | | JP 05588379 B2 | 10/09/2014 |
| | | JP 2006-500087 A | 05/01/2006 |
| | | JP 2006-500087 T | 05/01/2006 |
| | | JP 2010-000373 A | 07/01/2010 |
| | | JP 2011-078847 A | 21/04/2011 |
| | | JP 2011-136195 A | 14/07/2011 |
| | | JP 2014-131745 A | 17/07/2014 |
| | | KR 10-2005-0032108 A | 06/04/2005 |
| | | MX PA05001335 A | 08/09/2005 |
| | | US 2004-0024361 A1 | 05/02/2004 |
| | | US 2005-0038386 A1 | 17/02/2005 |
| | | US 2005-0038390 A1 | 17/02/2005 |
| | | US 2007-0250005 A1 | 25/10/2007 |
| | | US 2009-036771 A1 | 05/02/2009 |
| | | US 2011-184281 A1 | 28/07/2011 |
| | | US 6929619 B2 | 16/08/2005 |
| | | US 7632246 B2 | 15/12/2009 |
| | | US 7854726 B2 | 21/12/2010 |
| | | WO 2004-012787 A2 | 12/02/2004 |
| | | WO 2004-012787 A3 | 23/09/2004 |
| US 2011-0224644 A1 | 15/09/2011 | CN 102149417 A | 10/08/2011 |
| | | CN 102149417 B | 20/11/2013 |
| | | DK 2163273T3 | 29/07/2013 |

Form PCT/ISA/210 (patent family annex) (July 2009)

INTERNATIONAL SEARCH REPORT
Information on patent family members

International application No.
PCT/KR2014/001657

| Patent document cited in search report | Publication date | Patent family member | Publication date |
|---|---|---|---|
| | | DK 2361646T3 | 17/06/2013 |
| | | EP 2163273 A1 | 17/03/2010 |
| | | EP 2163273 B1 | 24/04/2013 |
| | | EP 2361646 A1 | 31/08/2011 |
| | | EP 2361646 B1 | 20/03/2013 |
| | | HK 1160616 A1 | 04/04/2014 |
| | | US 2011-0245781 A1 | 06/10/2011 |
| | | US 2013-0165899 A1 | 27/06/2013 |
| | | US 8469930 B2 | 25/06/2013 |
| | | US 8500700 B2 | 06/08/2013 |
| | | WO 2010-028719 A1 | 18/03/2010 |
| US 2009-0124974 A1 | 14/05/2009 | US 7850649 B2 | 14/12/2010 |

Form PCT/ISA/210 (patent family annex) (July 2009)